# EUROPEAN PATENT SPECIFICATION

(11) **EP 1 707 962 B1**
(45) Date of publication and mention of the grant of the patent: **23.05.2012**
(21) Application number: 05703967.9
(22) Date of filing: 21.01.2005
(51) Int. Cl.: G01N 33/52, G01N 33/543

(54) **SPECIMEN ANALYZING TOOL**
PROBENANALYSEWERKZEUG
OUTIL D'ANALYSE DE SPECIMENS

(30) Priority: 23.01.2004 JP 2004015740
(43) Date of publication of application: 04.10.2006
(73) Proprietor: ARKRAY, Inc., Kyoto-shi, Kyoto 601-8045 (JP)
(72) Inventor: SAKAMOTO, Hisashi, Kyoto-shi, Kyoto 601-8045 (JP); MURATA, Yasuhito, Kyoto-shi, Kyoto 601-8045 (JP)
(74) Representative: Hall, Matthew Benjamin
(86) International application number: PCT/JP2005/000739
(87) International publication number: WO 2005/071406

(56) References cited:
- EP-A- 0 538 830
- EP-A1- 0 475 692
- WO-A-2004/008146
- WO-A1-02/25283
- JP-A- 7 120 475
- JP-A- 2000 507 353
- US-A- 3 907 503
- US-A- 4 618 475
- US-A- 4 837 043
- US-A- 5 526 120
- US-B2- 6 514 769

## Description

### Technical Field

The present invention relates to a sample analysis tool.

### Background Art

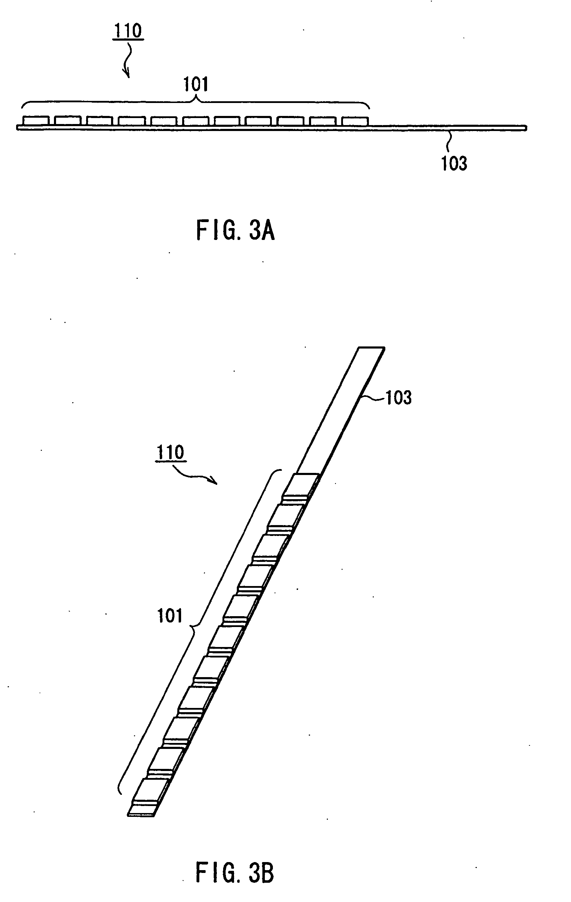

In clinical tests, a sample analysis tool in which a pad(s) impregnated with a reagent(s) is provided on a plastic substrate has been used for various purposes. An example of such a sample analysis tool is shown in FIG. 3. FIG. 3A is a front view of the sample analysis tool and FIG. 3B is a perspective view of the same. As shown in FIG. 3, in this sample analysis tool 110, eleven reagent pads 101 are arranged in series on a strip-shaped plastic substrate 103 along the longitudinal direction of the substrate 103. No regent pads are provided in one end portion of the substrate 103 (the end portion on the right in FIG. 3A), so that this portion serves as a holding portion to be pinched with fingers.
US 4,837,043 discloses a method of producing such test strips. Extruder casting machines are used to produce reagent zones on a common surface. The process permits application of matrix material not impregnated with reagent in order to create this common (flat) surface. US 4,618,475 discloses a test strip in which the reagent pads are separated from each other by hydrophobic barrier pads, once again forming a flat, common surface.

In such a sample analysis tool, reagents corresponding to test items are contained in the reagent pads, respectively. For example, in the case of urinalysis, the pads contain reagents for test items such as glucose (GLU), protein (PRO), bilirubin (BIL, urobilinogen (URO), pH, occult blood (BLD), ketone bodies (KET), nitrite (NIT), leukocytes (LEU), S.G. (specific gravity), and a color tone, respectively. These items can be tested by impregnating the respective pads of this sample analysis tool with urine and then measuring a change in color tone of each reagent either visually or with an optical analyzer. When measuring the change in color tone with an optical analyzer, a predetermined tube containing a urine sample is set in the analyzer together with the sample analysis tool. Then, an automatic feeder (see Patent Document 1, for example) automatically feeds the sample analysis tool to an analysis part, where the analysis of the sample analysis tool can be conducted automatically or semiautomatically. Note here that such a sample analysis tool also may be called a test piece or a test paper. However, in the case of a sample analysis tool for testing only two or three test items, the automatic feeding of the sample analysis tool set in the analyzer to the analysis part may not be achieved successfully.
Patent Document 1: JP 2000-035433 A

### Disclosure of Invention

### Problem to be Solved by the Invention

The present invention was made in light of the foregoing problem, and it is an object of the present invention to provide a sample analysis tool that can be fed to an analysis part of an analyzer automatically without causing any inconvenience when set in the analyzer, even in the case where the number of test items to be tested by the sample analysis tool is small.

### Means for Solving Problem

According to the present invention there is provided a sample analysis tool (100) comprising:
a substrate (103) in the shape of a strip where one end portion of the substrate serves as a holding portion and a portion other than the holding portion serves as a reagent pad arrangement region; and
at least one reagent pad (101), the at least one reagent pad being provided on a side opposite to the holding portion side in the reagent pad arrangement region;
wherein at least one balance pad (107) is provided on the holding portion side of the substrate so as to adjust weight balance of the sample analysis tool; and
the at least one reagent pad and the at least one balance pad are attached to the substrate with an adhesive or a pressure sensitive adhesive;
characterised in that the at least one reagent pad and the at least one balance pad are formed of a filter paper,
and in that the at least one balance pad is provided on the holding portion side of the substrate spaced apart from the at least one reagent pad.

### Effects of the Invention

The inventors of the present invention conducted studies to find the reasons why a sample analysis tool for testing a small number of test items cannot be fed to an analysis part of an analyzer successfully when it is set in the analyzer. As a result, the inventors found that the following is one cause of the failure in successful feeding of the sample analysis tool. The sample analysis tool for testing a small number of test items has a small number of reagent pads, and besides, these reagent pads are arranged so as to concentrate on one end portion of the substrate. Thus, the sample analysis tool lacks in weight balance and thus cannot be treated well by an automatic feeder or the like. Based on these findings, the inventors of the present invention discovered that, when a pad (containing no reagent) for adjusting weight balance of the sample analysis tool was provided on the substrate, the sample analysis tool could be treated well by the automatic feeder or the like so that it could be fed to the analysis part smoothly. Thus, the inventors of the present invention arrived at the present invention. Accordingly, the sample analysis tool of the present invention can be used in an analyzer without causing any inconvenience even in the case where the number of test items to be tested by the sample analysis tool is small. Thus, by the use of the sample analysis tools of the present invention, it becomes possible to treat a large number of samples efficiently.

### Brief Description of Drawings

[FIG. 1] FIG. 1 shows an example of a sample analysis tool of the present invention, wherein FIG. 1A is a front view of the sample analysis tool and FIG. 1B is a perspective view of the same.
[FIG. 2] FIG. 2 shows another example of a sample analysis tool of the present invention, wherein FIG. 2A is a front view of the sample analysis tool and FIG. 2B is a perspective view of the same.
[FIG. 3] FIG. 3 shows an example of a conventional sample analysis tool, wherein FIG. 3A is a front view of the sample analysis tool and FIG. 3B is a perspective view of the same.
[FIG. 4] FIG. 4 shows another example of a conventional sample analysis tool, wherein FIG. 4A is a front view of the sample analysis tool and FIG. 4B is a perspective view of the same.
[FIG. 5] FIG. 5 is an external perspective view showing an example of a test piece feeder.

### Explanation of letters or numerals

- 1: base
- 2: pole
- 3b: support member
- 4: rotating body
- 4e: groove
- 5: catching portion
- 6: test piece detection block
- 6e: partition plate
- 7: inclined cover
- 8: drum
- 9: base member
- 9b: arc-shaped portion
- 9c: discharging path
- 10: drive portion
- 10A: motor
- 10B: driving force transmitting system
- 11: storage portion
- 100: sample analysis tool
- 101: reagent pad
- 102: balance pad
- 103: substrate
- 104: pad arrangement region
- 105: holding portion
- 107: balance pad
- 110: sample analysis tool

### Best Mode for Carrying Out the Invention

A sample analysis tool according to a first embodiment of the present invention is configured so that: the substrate is in a strip shape; one end portion of the substrate serves as a holding portion and a portion other than the holding portion serves as a reagent pad arrangement region; the at least one reagent pad is provided on a side opposite to a holding portion side in the reagent pad arrangement region and the balance pad is provided on the holding portion side in the reagent pad arrangement region.

A sample analysis tool according to a second embodiment of the present invention is configured so that: the substrate is in a strip shape; one end portion of the substrate serves as a holding portion and a portion other than the holding portion serves as a reagent pad arrangement region; the at least one reagent pad is provided on a side opposite to a holding portion side in the reagent pad arrangement region and the balance pad is provided in the holding portion.

It is to be noted here that the sample analysis tool of the present invention is not limited to the above-described two embodiments.

In the first embodiment, the balance pad may be provided in the holding portion.

In the second embodiment, the balance pad may be provided in the reagent pad arrangement region.

Furthermore, in the present invention, the balance pad for adjusting the weight balance may be provided on a rear surface of the substrate, taking a surface of the substrate provided with the reagent pad as a front surface.

In the first embodiment and the second embodiment, in the case where two or more reagent pads are provided, the reagent pads may be arranged in series along the longitudinal direction of the strip-shaped substrate.

There is no particular limitation on the use of the sample analysis tool of the present invention. For example, the sample analysis tool of the present invention can be used for a general clinical test, an urinalysis, a biochemical test, a microorganism test, an immunological test, a genetic analysis, an environmental test, a test for an agricultural chemical, or an allergen test.

When the sample analysis tool of the present invention is used for an urinalysis, examples of a test item include glucose (GLU), protein (PRO), bilirubin (BIL), urobilinogen (URO), pH, occult blood (BLD), ketone bodies (KET), nitrite (NIT), leukocytes (LEU), S.G. (specific gravity), a color tone, ascorbic acid, a salt concentration, highly-sensitive protein, albumin, creatinine, Bence-Jones protein, hormones, and physiologically active substances. In the sample analysis tool of the present invention, at least one reagent pad is provided for each test item.

The sample analysis tool of the present invention preferably is analyzed by an analyzer, but the present invention is not limited thereto. For example, the sample analysis tool of the present invention may be subjected to visual observation.

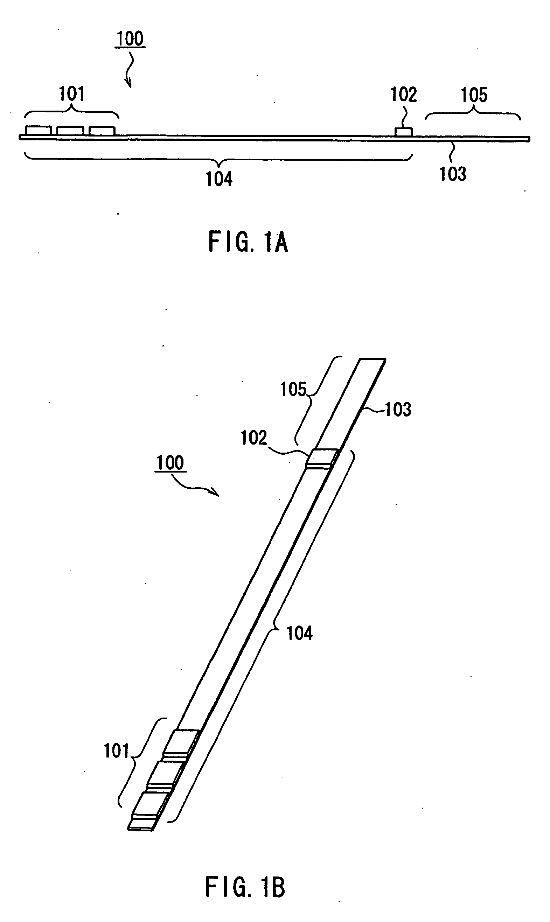

FIG. 1 shows an example (the first embodiment) of a sample analysis tool of the present invention. FIG. 1A is a front view of the sample analysis tool and FIG. 1B is a perspective view of the same.

As shown in FIG. 1, in this sample analysis tool 100, reagent pads 101 and a balance pad 102 are provided on a strip-shaped substrate 103. One end portion of the substrate 103 (the end portion on the right in FIG. 1A) serves as a holding portion 105 to be pinched with fingers, and the region other than the holding portion 105 serves as a reagent pad arrangement region 104. In this sample analysis tool 100, three reagent pads 101 are arranged in series at regular intervals along the longitudinal direction of the strip-shaped substrate 103 in an end portion of the reagent pad arrangement region 104 on a side opposite to the holding portion 105 side. Furthermore, one balance pad is provided on the holding portion 105 side in the reagent pad arrangement region 104 so as to adjust weight balance of the sample analysis tool 100 as a whole. In the sample analysis tool of the present invention, a marker for discriminating the front and rear surfaces of the substrate preferably is formed on the substrate, although such a marker is not shown in FIG. 1.

The material of the substrate is not particularly limited, and may be, for example, a resin, metal, glass, or the like. Also, the color of the substrate is not particularly limited, and may be white, gray, black, a chromatic color, or transparent. Furthermore, the size of the substrate is not particularly limited, and may be determined as appropriate depending on a test item, the standard of an analyzer to be used, etc. For example, when the substrate is in a strip shape, the substrate may be 50 to 150 mm in length, 2 to 10 mm in width, and 0.1 to 1.0 mm in thickness.

The reagent pad and the balance pad are formed of a filter paper. The shapes of the reagent pad and the balance pad are not particularly limited, and may be square, rectangular, circular, oval, or the like. The sizes of the reagent pad and the balance pad are not particularly limited. For example, when they are in a square shape, they may be 2 to 10 mm in vertical and horizontal lengths and 0.05 to 1.0 mm in thickness. The number of the reagent pads is not particularly limited, and may be, for example, in the range from 1 to 8. The reagent pad may be obtained by impregnating a pad material with a reagent and then forming the pad material into a predetermined shape or by forming a pad material into a predetermined shape and then impregnating the pad material with a reagent. The impregnation with the reagent can be achieved by, for example, immersing the pad material in a reagent solution and then drying the pad material. Furthermore, the pad is provided on the substrate by. using an adhesive or a pressure sensitive adhesive. Examples of the adhesive and the pressure sensitive adhesive include those based on polyurethane, acrylic substances, vinyl chloride, epoxy, nylon, hot melt, cyanoacrylate, rubbers, and the like.

The number of the balance pads is determined as appropriate depending on the number of the reagent pads, the positions of the reagent pads, etc. For example, when the reagent pads and the balance pad are arranged in the manner as shown in FIG. 1, it is preferable that one to three balance pads are provided with respect to one to eight reagent pads.

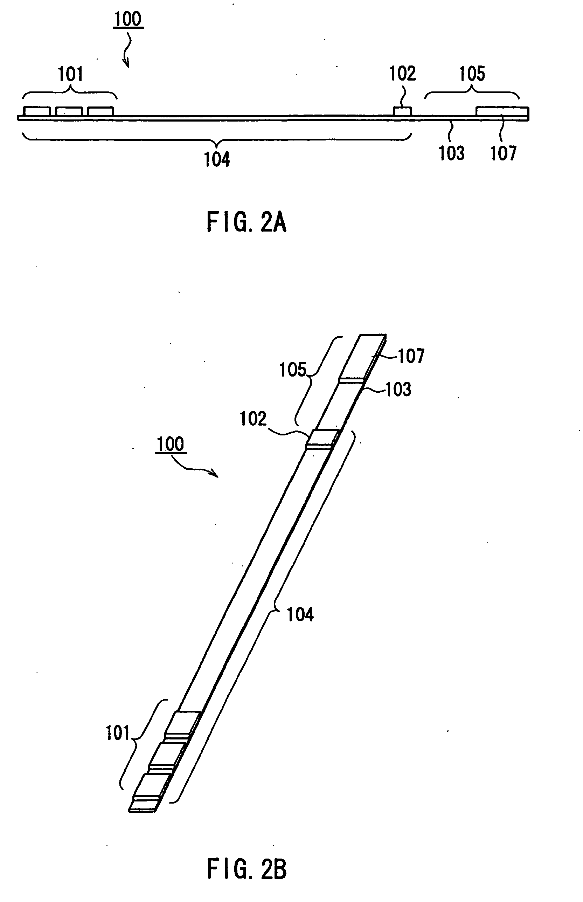

FIG. 2 shows another example (the second embodiment) of a sample analysis tool of the present invention. FIG. 2A is a side view of the sample analysis tool and FIG. 2B is a perspective view of the same. In FIG. 2, the same components as those in FIG. 1 are denoted with the same reference numerals as those therein.

As shown in FIG. 2, in the sample analysis tool according to this example, in addition to a first balance pad 102 in a reagent pad arrangement region 104, a second balance pad 107 is provided in a holding portion 105. By the use of these two balance pads, the weight balance of the sample analysis tool can be adjusted more correctly, so that the sample analysis tool can be fed more stably in an automatic analyzer. Although FIG. 2 shows an example where the second balance pad 107 is larger that the first balance pad 102, the present invention is not limited thereto. The sizes and the positions of the first and second balance pads may be determined, taking various conditions such as the size, number, and positions of the reagent pads into account. Although the two balance pads 102 and 107 were used in combination in the present example, the present invention is not limited thereto and may be embodied such that a balance pad is provided only in the holding portion 105. Except for the balance pad being provided in the holding portion 105, the configuration of the sample analysis tool according to this example, i.e., the material, the size, the position, and the number of the pads, etc., is the same as that of the sample analysis tool according to the above example (FIG. 1). Note here that, when the second balance pad 107 provided in the holding portion 105 is made of a material having a high friction coefficient, there is an advantageous effect that the sample analysis tool can be pinched with fingertips more easily when holding the sample analysis tool.

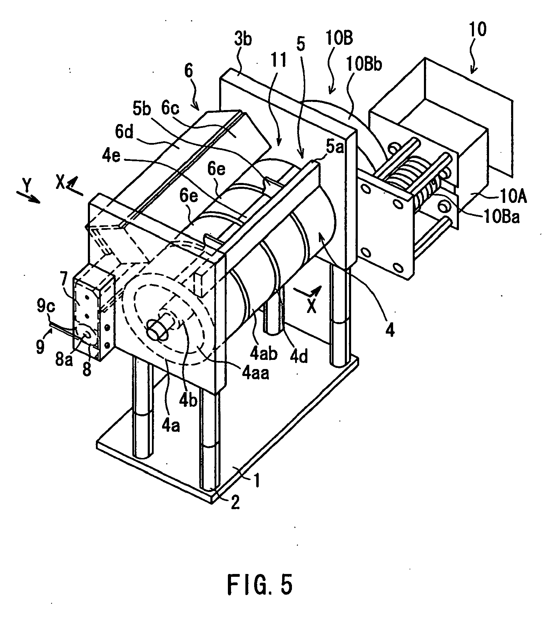

Next, an example of an automatic sample analysis tool (test piece) feeder of an analyzer to which the sample analysis tool of the present invention is applicable will be described with reference to FIG. 5.

FIG. 5 is an external perspective view showing an entire external appearance of an example of a sample analysis tool feeder to which a sample analysis tool (a test piece) of the present invention is applicable. The test piece feeder shown in FIG. 5 constitutes a part of a continuous test apparatus for examining test pieces for urinalysis or the like continuously and is provided for feeding the sample analysis tools (the test pieces) to a predetermined examination portion of the continuous test apparatus one by one successively.

As shown in FIG. 5, this test piece feeder is configured schematically so as to include a base 1, poles 2, support members 3b, a rotating body 4, a catching portion 5, a test piece detection block 6, an inclined cover 7, a drum 8, a base member 9, a drive portion 10, and a drum control unit. The drum control unit is composed of a microcomputer and the like. Since the drum control unit does not have a mechanical configuration and does not provide any mechanical actions, it is not shown in the drawing.

The base 1 and the poles 2 serve to lift the support members 3b above the installation surface. The support members 3b, the catching portion 5, and the test piece detection block 6 form a side wall surrounding a storage portion 11 in which a large number of test pieces are supplied and stored. The outer periphery of the rotating body 4 blocks the opening of the storage portion 11, thus serving as the bottom of the storage portion 11. The rotating body 4 serves as a test piece feeding mechanism for taking out test pieces from the storage portion 11 one by one and feeding them to the drum 8. Inside the test piece detection block 6, a photosensor that is not shown in FIG. 5 is incorporated. This sensor detects whether a single test piece fed from the rotating body 4 to the drum 8 faces up or down. Since the test piece slides down from the rotating body 4 to the drum 8, the inclined cover 7 and the base member 9 form an inclined path that is not shown in FIG. 5. In the drive portion 10, a motor 10A for impart a turning force to the rotating body 4 and the drum 8 is incorporated. The driving shaft of the motor 10A is connected to the rotation axes of the rotating body 4 and the drum 8 via a driving force transmitting system 10B including a belt and pulleys 10Ba and 10Bb. Note here that some parts of the driving force transmitting system are not shown in the drawing. This driving force transmitting system 10B, which will be described more specifically later, is divided into a driving force transmitting system for the rotating body and a driving force transmitting system for the drum. This driving force transmitting system for the drum and a drum control unit that will be described later composes a drum reversing mechanism.

Next, by taking the sample analysis tool automatic feeder shown in FIG. 5 as an example, how the inventors of the present invention arrived at the present invention will be described.

As shown in FIG. 5, as a mechanism for automatically taking out a test piece, a groove 4e to which only a test piece(s) can be inserted is provided in the rotating body 4. Test pieces placed on the top face of the rotating body 4 are inserted into the groove 4e one by one when the rotating body 4 is rotated clockwise and counterclockwise alternately (hereinafter such a rotation is referred to as "reciprocating rotation"). Thus, the test pieces can be taken out. The test piece inserted in the groove 4e of the rotating body 4 passes through the test piece detection block 6. However, when two overlapping test pieces are in the groove 4e, a partition plate 6e provided at the entrance of the test piece detection block 6 prevents the upper test piece from entering the test piece detection block 6. After the test piece has entered the test piece detection block 6, the photosensor detects whether the test piece faces up or down. Then, the test piece is subjected to a subsequent inverting step, in which test pieces facing down are inverted so that all the test pieces face up. Thereafter, the test piece is fed to a urine dropping unit for a subsequent step, in which a predetermined amount of urine is dropped on each test piece and spectrochemical analysis is performed automatically with respect to each test piece. However, the automatic feeder using the rotating body and the drum has the following two problems.

One problem is as follows. In a sample analysis tool (a test piece) as shown in FIG. 3, when nine to eleven reagent pads are attached to the substrate 103, it is possible to take out the test piece efficiently by reciprocatingly rotating the rotating body 4 a few times. However, when the number of the reagent pads is 8 or less, the test piece cannot be taken out even if the rotating body 4 is rotated reciprocatingly ten-odd times.

The other problem is as follows. In the case where a sample analysis tool (a test piece) as shown in FIG. 3 includes eight or less reagent pads, when the test piece is inserted in the groove 4e with the test piece facing up (i.e., with the surface provided with the reagent pads facing up), a large space is provided between the partition plate 6e and the test piece. Thus, the holding portion 105 of another test piece may be lodged in this space.

Accordingly, the inventors of the present invention investigated the cause that hinders the smooth insertion of a test piece in the groove 4e. As a result, the inventors found that the weight of the test piece (see FIG. 3) was lacking in balance and concentrated on the side opposite to the holding portion 105 side (i.e., the reagent pad side) so that, during the reciprocating rotation of the rotating body 4, only the side of the test piece with a heavier weight (the reagent pad side) could move on the surface of the rotating body 4 whereas the holding portion 105 side slid over the surface of the rotating body. In particular, when the test piece faced down (i.e., when the surface provided with the reagent pads faced the rotating body), only the reagent pads of the test piece were in contact with the rotating body 4, so that the resistance between the holding portion 105 and the surface of the rotating body 4 became small. This prevented the test piece from being in parallel with the groove 4e, thereby hindering the smooth insertion of the test piece into the groove 4e.

In view of the above, it was found that tasks to be addressed were preventing the test piece from being tilted with respect to the groove 4e during the reciprocating rotation of the rotating body 4 and eliminating the space between the partition plate 6e and the groove 4e. Thus, according to the present invention, a balance pad is used (e.g., attached on the holding portion 105 side) in a test piece for testing a small number of test items of, for example, eight or less. This allows the test piece to be taken out stably and efficiently, thus solving the above-described two problems.

In general, both the front and rear surfaces of the main body (substrate) of the test piece assume white as a base color. However, in order to allow the photosensor to detect whether the test piece faces up or down accurately as described above, it is preferable that a marker is formed in a portion other than the portions to which the reagent pads are attached on the surfaces of the main body. Examples of the marker include a black marker.

Hereinafter, examples of the present invention will be described along with comparative examples

### Example 1

Sample analysis tools (test pieces) provided with three reagent pads and one balance pad as shown in FIG. 1 and sample analysis tools (test pieces) provided with three reagent pads and two balance pads as shown in FIG. 2 were produced. Then, ten test pieces per each type were placed on the rotating body of the test piece feeder shown in FIG. 5, and the rotating body was rotated reciprocatingly until all the test piece were taken out. The number of rotations of the rotating body was measured. The results are shown in Table 1 below.

**[Table 1]**

| Test piece No. | three reagent pads + one balance pad | three reagent pads + two balance pads |
|---|---|---|
| | number of reciprocating rotations | number of reciprocating rotations |
| 1 | 4 | 2 |
| 2 | 3 | 1 |
| 3 | 2 | 1 |
| 4 | 2 | 2 |
| 5 | 3 | 2 |
| 6 | 1 | 3 |
| 7 | 2 | 1 |
| 8 | 2 | 2 |
| 9 | 3 | 2 |
| 10 | 3 | 3 |

### (Comparative Example 1)

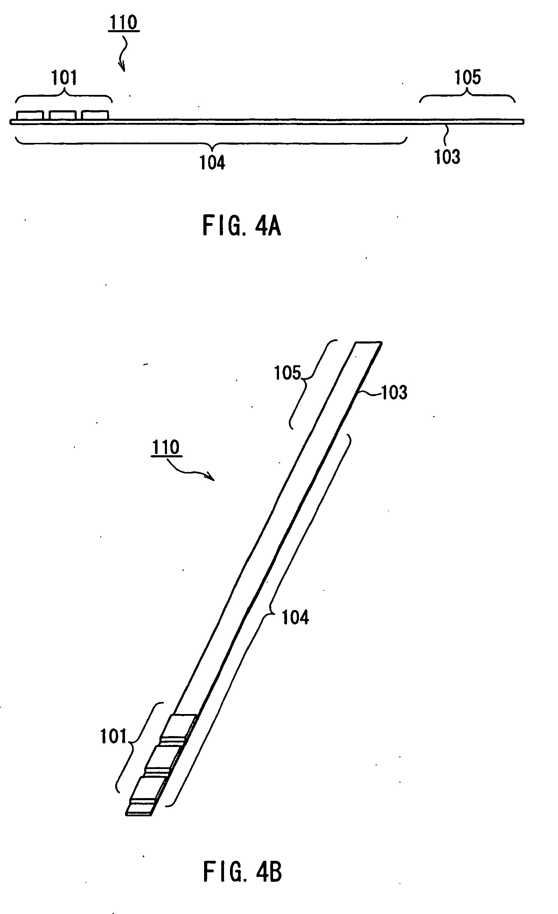

Test pieces provided with eleven reagent pads as shown in FIG. 3 and test pieces provided with only three reagent pads as shown in FIG. 4 were produced, and measurement using these test pieces was carried out in the same manner as in Example 1. The results are shown in Table 2 below.

**[Table 2]**

| Test piece No. | eleven reagent pads | three reagent pads |
|---|---|---|
| | number of reciprocating rotations | number of reciprocating rotations |
| 1 | 2 | 11 |
| 2 | 3 | 8 |
| 3 | 3 | 9 |
| 4 | 1 | clogged |
| 5 | 2 | 10 |
| 6 | 3 | 9 |
| 7 | 2 | 7 |
| 8 | 1 | 16 |
| 9 | 2 | 11 |
| 10 | 2 | 7 |

As shown in Table 1 and Table 2, in the case where many reagent pads were provided, no significant differences were observed between the example and the comparative example. However, in the case where a small number of reagent pads were provided, the number of reciprocating rotations required for taking out all the sample analysis tools (the test pieces) of the examples was only 1 to 3, which was much smaller than that required for taking out all the sample analysis tools (the test pieces) of the comparative example. The number of reciprocating rotations required for taking out all the test pieces of the comparative example was as great as 7 to 11 and besides, the occurrence of clogging was observed. From these results, it can be said that, by providing a balance pad, even a test piece for testing a small number of test items can be taken out with a significantly improved probability and besides, the clogging can be prevented from occurring so that the test piece can be taken out stably.

### Industrial Applicability

There is no particular limitation on the use of a sample analysis tool of the present invention. The sample analysis tool of the present invention can be used, for example, in clinical tests, biochemical tests, etc., but is particularly suitable for clinical tests that require a large number of samples to be processed with an analyzer.

## Claims

1. A sample analysis tool (100) comprising:
a substrate (103) in the shape of a strip where one end portion of the substrate serves as a holding portion and a portion other than the holding portion serves as a reagent pad arrangement region; and
at least one reagent pad (101), the at least one reagent pad being provided on a side opposite to the holding portion side in the reagent pad arrangement region;
wherein at least one balance pad (107) is provided on the holding portion side of the substrate so as to adjust weight balance of the sample analysis tool; and
the at least one reagent pad and the at least one balance pad are attached to the substrate with an adhesive or a pressure sensitive adhesive;
**characterised in that** the at least one reagent pad and the at least one balance pad are formed of a filter paper,
and **in that** the at least one balance pad is provided on the holding portion side of the substrate spaced apart from the at least one reagent pad.

2. The sample analysis tool (100) according to claim 1, wherein the number of reagent pads (101) is in a range from 1 to 8 and the number of balance pads (107) is in a range of 1 to 3.

3. The sample analysis tool (100) according to claim 2, comprising one, two or three reagent pads (101).

4. The sample analysis tool (100) according to claim 3, comprising 3 reagent pads (101).

5. The sample analysis tool (100) according to any preceding claim, wherein the at least one balance pad (107) is provided on the holding portion side of the substrate in the reagent pad arrangement region (104) and/or in the holding portion (105).

6. The sample analysis tool (100) according to any preceding claim, wherein the at least one balance pad/s (107) is/are pads containing no reagent.

7. The sample analysis tool (100) according to any preceding claim, wherein two or more reagent pads (101) are arranged in series along a longitudinal direction of the strip-shaped substrate (103).

8. The sample analysis tool (100) according to any preceding claim, which is used for an urinalysis, a biochemical test, a microorganism test, an immunological test, a genetic analysis, an environmental test, a test for an agricultural chemical, or an allergen test.

9. The sample analysis tool (100) according to claim 7, wherein a test item in the urinalysis is at least one selected from the group consisting of glucose (GLU), protein (PRO), bilirubin (BIL), urobilinogen (URO), pH, occult blood (BLD), ketone bodies (KET), nitrite (NIT), leukocytes (LEU), S.G. (specific gravity), a color tone, ascorbic acid, a salt concentration, highly-sensitive protein, albumin, creatinine, Bence-Jones protein, hormones, and physiologically active substances, and at least one reagent pad (101) is provided for each test item.

10. The sample analysis tool according to any preceding claim, which is analyzed by an analyzer.

## Patentansprüche

1. Probenanalysewerkzeug (100), das enthält:
ein Substrat (103) in Form eines Streifens, wobei ein Endabschnitt des Substrats als Halteabschnitt dient und ein von dem Halteabschnitt verschiedener Abschnitt als Reagenzfeld-Anordnungsbereich dient; und
mindestens ein Reagenzfeld (101), wobei das mindestens eine Reagenzfeld auf einer zur Halteabschnittsseite entgegengesetzten Seite im Reagenzfeld-Anordnungsbereich vorgesehen ist;
wobei mindestens ein Ausgleichsfeld (107) auf der Halteabschnittsseite des Substrats vorgesehen ist, um den Gewichtsausgleich des Probenanalysewerkzeugs einzustellen; und
das mindestens eine Reagenzfeld und das mindestens eine Ausgleichsfeld am Substrat mit einem Klebstoff oder einem druckempfindlichen Klebstoff befestigt sind;
**dadurch gekennzeichnet, dass** das mindestens eine Reagenzfeld und das mindestens eine Ausgleichsfeld aus einem Filterpapier ausgebildet sind,
und dass das mindestens eine Ausgleichsfeld auf der Halteabschnittsseite des Substrats von dem mindestens einen Reagenzfeld beabstandet vorgesehen ist.

2. Probenanalysewerkzeug (100) nach Anspruch 1, wobei die Anzahl von Reagenzfeldern (101) in einem Bereich von 1 bis 8 liegt und die Anzahl von Ausgleichsfeldern (107) in einem Bereich von 1 bis 3 liegt.

3. Probenanalysewerkzeug (100) nach Anspruch 2 mit einem, zwei oder drei Reagenzfeldern (101).

4. Probenanalysewerkzeug (100) nach Anspruch 3 mit 3 Reagenzfeldern (101).

5. Probenanalysewerkzeug (100) nach einem vorangehenden Anspruch, wobei das mindestens eine Ausgleichsfeld (107) auf der Halteabschnittsseite des Substrats im Reagenzfeld-Anordnungsbereich (104) und/oder im Halteabschnitt (105) vorgesehen ist.

6. Probenanalysewerkzeug (100) nach einem vorangehenden Anspruch, wobei das mindestens eine Ausgleichsfeld (107) (ein) Ausgleichsfeld(er) ist(sind), das (die) kein Reagenz enthält (enthalten).

7. Probenanalysewerkzeug (100) nach einem vorangehenden Anspruch, wobei zwei oder mehr Reagenzfelder (101) in Reihe entlang einer Längsrichtung des streifenförmigen Substrats (103) angeordnet sind.

8. Probenanalysewerkzeug (100) nach einem vorangehenden Anspruch, das für eine Urinanalyse, einen biochemischen Test, einen Mikroorganismentest, einen immunologischen Test, eine Genanalyse, einen Umwelttest, einen Test für eine landwirtschaftliche Chemikalie oder einen Allergentest verwendet wird.

9. Probenanalysewerkzeug (100) nach Anspruch 7, wobei ein Testelement in der Urinanalyse mindestens eines ist, das aus der Gruppe ausgewählt ist, die aus Glucose (GLU), Protein (PRO), Bilirubin (BIL), Urobilinogen (URO), pH, okkultem Blut (BLD), Ketonkörpern (KET), Nitrit (NIT), Leukozyten (LEU), S.G. (spezifischem Gewicht), einem Farbton, Ascorbinsäure, einer Salzkonzentration, einem hochempfindlichen Protein, Albumin, Kreatinin, einem Bence-Jones-Protein, Hormonen und physiologisch aktiven Substanzen besteht und mindestens ein Reagenzfeld (101) für jedes Testelement vorgesehen ist.

10. Probenanalysewerkzeug nach einem vorangehenden Anspruch, das durch einen Analysator analysiert wird.

## Revendications

1. Outil d'analyse d'échantillon (100) comportant :
un substrat (103) ayant la forme d'une bande où une partie d'extrémité du substrat sert de partie de retenue et une partie autre que la partie de retenue sert de région d'agencement de tampon de réactif ; et
au moins un tampon de réactif (101), le au moins un tampon de réactif étant disposé sur un côté opposé au côté de la partie de retenue dans la région d'agencement de tampon de réactif ;
dans lequel au moins un tampon d'équilibrage (107) est disposé du côté de la partie de retenue du substrat de manière à équilibrer le poids de l'outil d'analyse d'échantillon ; et
le au moins un tampon de réactif et le au moins un tampon d'équilibrage sont fixés au substrat par un adhésif ou un adhésif sensible à une pression ;
**caractérisé en ce que** le au moins un tampon de réactif et le au moins un tampon d'équilibrage sont formés d'un papier filtrant,
et **en ce que** le au moins un tampon d'équilibrage est disposé du côté de la partie de retenue du substrat espacé du au moins un tampon de réactif.

2. Outil d'analyse d'échantillon (100) selon la revendication 1, dans lequel le nombre de tampons de réactif (101) est dans une plage de 1 à 8 et le nombre de tampons d'équilibrage (107) est dans une plage de 1 à 3.

3. Outil d'analyse d'échantillon (100) selon la revendication 2, comportant un, deux ou trois tampons de réactif (101).

4. Outil d'analyse d'échantillon (100) selon la revendication 3, comportant 3 tampons de réactif (101).

5. Outil d'analyse d'échantillon (100) selon l'une quelconque des revendications précédentes, dans lequel le au moins un tampon d'équilibrage (107) est disposé du côté de la partie de retenue du substrat dans la région d'agencement de tampon de réactif (104) et/ou dans la partie de retenue (105).

6. Outil d'analyse d'échantillon (100) selon l'une quelconque des revendications précédentes, dans lequel le au moins un tampon d'équilibrage (107) est/sont des tampons ne contenant aucun réactif.

7. Outil d'analyse d'échantillon (100) selon l'une quelconque des revendications précédentes, dans lequel deux ou plus de deux tampons de réactif (101) sont disposés en série le long d'une direction longitudinale du substrat en forme de bande (103).

8. Outil d'analyse d'échantillon (100) selon l'une quelconque des revendications précédentes, lequel est utilisé pour une analyse d'urine, un essai biochimique, un essai sur des microorganismes, un essai immunologique, une analyse génétique, un essai environnemental, un essai pour un produit chimique agricole, ou un essai d'allergène.

9. Outil d'analyse d'échantillon (100) selon la revendication 7, dans lequel un élément d'essai dans l'analyse d'urine est au moins un élément choisi parmi le groupe constitué de glucose (GLU), d'une protéine (PRO), de bilirubine (BIL), d'urobilinogène (URO), d'un pH, d'une hémorragie occulte (BLD), de corps cétoniques (KET), d'un nitrite (NIT), de leucocytes (LEU), de S.G. (poids spécifique), d'un ton de couleur, d'acide ascorbique, d'une concentration de sels, d'une protéine fortement sensible, d'albumine, de créatinine, d'une protéine de Bence-Jones, d'hormones et de substances physiologiquement actives, et au moins un tampon de réactif (101) est prévu pour chaque élément d'essai.

10. Outil d'analyse d'échantillon selon l'une quelconque dés revendications précédentes, qui est analysé par un analyseur.
